Europäisches Patentamt

European Patent Office

Office européen des brevets

(11) Publication number: **0 064 103**
**B1**

(12) **EUROPEAN PATENT SPECIFICATION**

(45) Date of publication of patent specification: **06.11.85**

(21) Application number: **81301877.7**

(22) Date of filing: **28.04.81**

(51) Int. Cl.⁴: **A 61 K 39/40, A 61 K 39/395**

(54) Method of obtaining an anti-inflammatory bovine milk.

(43) Date of publication of application:
**10.11.82 Bulletin 82/45**

(45) Publication of the grant of the patent:
**06.11.85 Bulletin 85/45**

(84) Designated Contracting States:
**AT BE CH DE FR GB IT LI LU NL SE**

(56) References cited:
**FR-A-1 599 671
GB-A-1 211 876
GB-A-1 505 513
GB-A-1 573 995
GB-A-2 013 691
US-A-3 128 230
US-A-3 376 198**

(73) Proprietor: **THE STOLLE RESEARCH AND
DEVELOPMENT CORPORATION
6990 Cornell Road
Cincinnati Ohio 45242 (US)**

(72) Inventor: **Beck, Lee R.
2550 Dunmore Place
Birmingham Alabama 35226 (US)**

(74) Representative: **Burford, Anthony Frederick
et al
W.H. Beck, Greener & Co. 7 Stone Buildings
Lincoln's Inn
London WC2A 3SZ (GB)**

Courier Press, Leamington Spa, England.

# 0 064 103

**Description**

The present invention relates to anti-inflammatory milk and provides a process for its production. Inflammation, as defined in Dorland's Medical Dictionary, is "a localized protective response elicited by injury or destruction of tissues which serves to destroy, dilute or wall off (sequester) both the injurious agent and the injured tissue. It is characterized in the acute form by the classical sequence of pain (dolor), heat (calor), redness (rubor), swelling (tumor), and loss of function (functio laesa). Histologically, it involves a complex series of events including dilation of the arterioles, capillaries, and venules with increased permeability and blood flow; exudation of fluids, including plasma proteins; and leukocytic migration into the inflammatory focus".

The inflammatory response is any response characterized by inflammation as defined above. It is well known to those skilled in the medical arts that the inflammatory response causes much of the physical discomfort, i.e., pain and loss of function, that has come to be associated with different diseases and injuries. Accordingly, it is a common medical practice to administer pharmacological agents which have the effect of neutralizing the inflammatory response. Agents having these properties are classified as antiinflammatory drugs. Anti-inflammatory drugs are used for the treatment of a wide spectrum of disorders and the same drugs are often used to treat different diseases. Treatment with anti-inflammatory drugs is not for the disease but rather for the symptom, i.e., inflammation.

Corticosteroids represent the most widely used class of compounds for the treatment of the anti-inflammatory response. Proteolytic enzymes represent another class of compounds which are claimed to have anti-inflammatory effects. Hormones which directly or indirectly cause the adrenal cortex to produce and secrete steroids represent another class of anti-inflammatory compounds. A number of non-hormonal anti-inflammatory agents have been described. Examples of steroidal and non-steroidal anti-inflammatory agents are listed in the Physician's Desk Reference to Pharmaceuticals, Specialities, and Biologicals, 1979.

The natural and synthetic corticosteroid preparations cause a number of severe side effects including elevation of blood pressure, salt and water retention, and increased potassium and calcium excretion. Moreover, corticosteroids may mask the signs of infection and enhance dissemination of infectious microorganisms. These hormones are not considered safe for use in pregnant women, and long-term corticosteroid treatment has been associated with gastric hyperactivity and/or peptic ulcers. Treatment with these compounds may also aggravate diabetes mellitus, requiring higher doses of insulin, and psychotic disorders. Hormonal anti-inflammatory agents which act indirectly to increase the production of endogenous corticosteroids have the same potential for adverse side effects. The non-hormonal anti-inflammatory agents are synthetic biochemical compounds which can be toxic at high doses with a wide spectrum of undesirable side effects. Accordingly, in spite of the large number of anti-inflammatory agents that are currently available, there still exists a need for a safe, effective, anti-inflammatory product which is free of side effects and adverse reactions.

If a natural food product, such as milk for example, could be obtained having anti-inflammatory effects it would be an easily administerable, readily available, safe therapeutic composition.

It has been known in the prior art to produce milks having a variety of therapeutic effects. The present inventor for example has disclosed a milk containing antibodies to *Streptococcus mutans* which has dental caries inhibiting effects (*Beck*, GB—A—1,505,513). The milk is obtained by immunizing a cow with *S. mutans* antigen in two stages and obtaining the therapeutic milk therefrom. The present inventor has also disclosed a milk having antiarthritic properties (U.K. Patent Specification No. 2013691 published 15th August 1979). *Heinbach*, U.S. Patent 3,128,230, has described milk containing globulins of α, β and γ components, obtained by inocculating a cow with antigenic mixtures. *Petersen* (U.S. Patent 3,376,198 and Canadian Patent 587,849), *Holm*, U.S. Application (published) SN 628,987 and *Tunnak et al* (British Patent 1,211,876) have also described antibody-containing milks. None of the aforementioned references however disclose or suggest milk having anti-inflammatory properties.

According to a first aspect of the present invention, there is provided a method of obtaining an anti-inflammatory milk or anti-inflammatory product derived therefrom which comprises:—

selecting an antigen or mixture of antigens capable of inducing a state of immune sensitivity in a bovid,

sensitizing the bovid by primary immunization with said antigen(s),

testing the serum of the bovid to confirm sensitivity induction, and

administering to the bovid booster doses of said antigen(s) on a periodic basis to maintain a hyperimmune state,

characterised in that milk produced by the bovid is tested for anti-inflammatory activity and milk is collected for the purposes of the method from the bovid only during the period said test confirms that the milk contains an anti-inflammatory factor.

According to the second aspect of the present invention, there is provided a method of obtaining an anti-inflammatory milk or an anti-inflammatory product derived therefrom which comprises:—

testing milk from a bovid being maintained in a hyperimmune state and

collecting milk from the bovid during said hyperimmune state,

characterised in that milk produced by the bovid is tested for anti-inflammatory activity and the milk is

2

collected for the purpose of the method from the bovid only during the period said test confirms that the milk contains an anti-inflammatory factor.

According to a third aspect of the present invention, there is provided a method of obtaining an anti-inflammatory milk or anti-inflammatory product derived therefrom which comprises:—

selecting an antigen or mixture of antigens capable of inducing a state of immune sensitivity in a bovid, sensitizing the bovids by primary immunization with said antigen(s),

testing the serum of the bovids to confirm sensitivity induction, and

administering to the bovids booster doses of said antigen(s) on a periodic basis to maintain a hyperimmune state,

characterised in that milk produced by the bovid is tested for anti-inflammatory activity, bovids giving milk failing said anti-inflammatory test are subjected to readministration of said booster doses or rejected for the purposes of the method, and milk is collected in the method from the bovid only during the period said test confirms that the milk contains an anti-inflammatory factor.

The method of the invention provides a natural food product (milk) which has anti-inflammatory properties. The invention represents a significant advance in the state of the art of anti-inflammatory pharmacology because the product has no adverse side effects. Anti-inflammatory milk can be used to treat inflammation associated with any disease or injury in animals and humans without fear of side effects because it is a natural food product. Examples of human disease conditions which may be treated with anti-inflammatory milk include: rheumatoid arthritis; psoriatic arthritis; ankylositing spondylitis; acute and subacute bursitis; acute non-specific tendonitis; acute gouty arthritis, systemic lupus erythematosus; systemic dermatomyositis; acute rheumatic carditis; pemphigus; bullous dermatitis; hepetiformis; severe erythema; multiform exfoliative dermatitis, and cirrhosis, seasonal perennial rhinitis; bronchial asthma; contact dermatitis; etopic dermatitis; serum sickness; allergic conjunctivitis; deratitis; herpes zoster; opthalmicus iritis; diffuse uveitis, and choriditis; optic neuritis, and sympathetic opthalmia, symptomatic sarcoidosis, Loeffler's Syndrome, and berylliosis; hemolytic anemia; palliative management of neoplastic diseases including: leukemia, lymphomas, tuberculosis, and meningitis. It should be emphasized in this regard that the product does not treat the disease, but rather the symptom of the disease. Accordingly, the product is applicable for use in any disease or injury which exhibits inflammation as a symptom.

In the process of this invention, the bovid includes any milk-producing member of the genus *Bos*, preferable cows, sheep and goats, most preferably cows.

The invention is based on the discovery that when a bovid is brought to a specific state of immunization by means of periodic booster administrations of an antigen or a mixture of antigens, the bovid will produce milk which has the highly beneficial property of decreasing inflammatory conditions. The effect is caused by the presence of a factor in the milk, herein called "anti-inflammatory factor". The factor is not produced by all bovids that are immunized. The induction of immune sensitivity alone is insufficient to cause the appearance of anti-inflammatory factor in milk, as is shown by the fact that normal cow's milk does not contain this factor, even though cows have become sensitized against various antigens during normal immunization against cow diseases.

Furthermore, the factor is not always produced by bovids maintained in the immune state by booster injections. It is only in a specific hyperimmune state called herein "anti-inflammatory factor producing state" that the milk has the desired effects. This special state is only achieved by administering periodic boosters with sufficiently high doses of antigens or mixtures of antigens. These doses are herein called "anti-inflammatory-factor producing doses". The preferred dose range should be equal to or greater than 50% of the dosage necessary to cause primary sensitization of the bovid. Thus, there is a booster dosage threshold below which no factor is produced in the milk even though the cow may be in what is normally called an immune state. In order to achieve the anti-inflammatory factor producing state it is essential to test the bovid's milk after a first series of booster administrations. If the milk does not contain the factor, a second series of boosters of higher dosage has to be administered. This process is repeated until factor appears in the milk.

In summary the process comprises the following steps:
1. Antigen selection.
2. Sensitization of the bovid by primary immunization.
3. Testing the serum of the bovid to confirm sensitivity induction.
4. Administering boosters of appropriate dosage to induce and maintain an anti-inflammatory factor producing state.
5. Testing anti-inflammatory properties of milk.
6. Collecting milk from the bovid during the anti-inflammatory factor producing state.

Step 1—Any antigens or combination of antigens may be employed. The antigens can be bacterial, viral, cellular, or any other substances to which the immune system of a bovid will respond. The critical point in Step 1 is that the antigen must be capable of inducing a state of immune sensitivity in the cow. The antigen can be administered by any method which causes sensitization. Preferably polyvalent antigens are used.

Step 2—The preferred method of immunization is by intramuscular injection. However, other methods such as intravenous injection, intraperineal injection, oral administration, rectal suppository, can be used, providing the dose is sufficient to induce sensitivity. The dosage is normally $1 \times 10^6$ cells to $1 \times 10^{20}$ cells, preferably $10^8$ cells to $10^{10}$ cells, most preferably $2 \times 10^8$ cells.

Step 3 is to determine whether or not the cow has become sensitive to the antigen. There are a number of methods known to those skilled in the art of immunology to test for sensitivity, (Methods in Immunology and Immuno-Chemistry, William, C. A., Chase, W. M. Academic Press, N.Y., London (vols. 1—5) (1977)). Examples of these include skin sensitivity tests, serum tests for the presence of antibodies to the stimulating antigens, and tests designed to evaluate the ability of immune cells from the host to respond to the antigen. The type of test employed will depend to a large extent on the nature of the antigen used. The preferred method is to use a polyvalent vaccine consisting of multiple bacterial species as the antigen and to test for the presence of agglutinating antibodies in the serum of the cow before and after challenge with the vaccine. The appearance of milk antibodies after immunization with the vaccine is indicative of sensitivity, and at this point it is possible to proceed to Step 4. The minimum dose of antigen necessary to induce sensitivity depends on the type of antigen used.

Step 4 involves the induction and maintenance of the anti-inflammatory factor producing state. Once a bovid has been shown to be sensitized, this state is induced by repeated booster administrations of an appropriate dosage at fixed time intervals. The spacing of the administration depends on the nature of the antigen. A two-week booster interval is optimal for polyvalent bacterial antigens. Moreover, the booster administrations must not induce a state of immune tolerance. This will cause the animal to pass from an anti-inflammatory factor producing state to a state of immune tolerance to the antigen in which case the animal will cease to produce the anti-inflammatory factor.

It might also be possible, for example, to use a combination of different immunization procedures, i.e., intramuscular injection for primary immunization and intravenous injection for booster injections, etc. Many different combinations of immunization methods might be employed by those skilled in the art to: (1) sensitize and (2) induce the anti-inflammatory factor producing state.

Step 5 is to test the anti-inflammatory properties of the milk. The rat paw test is the standard animal test for anti-inflammatory drugs. ((1) Winter, C. A., Risley, G. A., Nuss, G. W., "Carrageenin-Induced Edema in the Hind Paw of the Rat as an Assay for Anti-inflammatory Drugs", Proc. Soc. Exp. Biol. Med. *3*, 544—547 (1967)). A variety of the other tests might be employed. ((2) Watnick, A. S., and Sabin, C., (1972) "The Effects of Clonixin and Bethamethasone on Adjuvant-Induced Arthritis and Experimental Allergic Encephalomyelitis in Rats"., Jap. J. Pharm. *22*, 741—748). However, the rat paw test is the most simple and direct test available, and has been shown to be highly satisfactory for all anti-inflammatory drugs. This test involves the injection of carrageenan in a small quantity into the foot pad of adult white rats. This is known to induce the inflammatory response. Within hours after treatment the inflammatory response causes the rat's foot to swell in size. The degree of swelling can be determined volumetrically and/or by determining the change in weight of the rat's paw. Anti-inflammatory drugs have the ability to block inflammation of the rat paw.

Step 6 involves collection and processing of the milk. The milk can be collected by conventional methods; however, special processing is necessary to protect the anti-inflammatory properties of the milk. The anti-inflammatory agent is heat sensitive. Accordingly, low temperature pasteurization is required. The pasteurization temperature should not exceed 140°C. Following pasteurization, the fat is removed by standard procedures and the milk is spray dried. Conventional spray-drying procedures are used, with exception that the milk is concentrated under vacuum at low temperature so as not to destroy the anti-inflammatory factor. (See e.g. Kosikowski, F., "Cheese and Fermented Milk Products", 2d Ed., 1977). The final product is a milk powder which has anti-inflammatory properties.

Fluid milk can also be used of course, as well as concentrated milk products or a fraction of the milk containing the biologically active factor such as the acid whey fraction.

The milk can be provided in any amount which affects the decrease of inflammatory conditions in warm-blooded animals. Daily amounts of 1 ml to 10 liters based on fluid milk can be provided, depending on the particular circumstance of the inflammation and the animal species.

The fat-free milk can of course be incorporated into any food product as long as the food product is not treated at a temperature which is too elevated and would inactivate the anti-inflammatory properties of the product. A temperature lower than 150°C is preferred. For example puddings or yoghurt may be prepared with anti-inflammatory milk.

Further, when the fat free milk is treated with acid at about room temperature (bringing the pH of the milk to about 4.2—4.6) and the casein is separated after precipitation thereof, it is found that the acid whey supernatant fraction contains the anti-inflammatory factor. This acid whey fraction may also be added to syrups, ice-cream mixes, candy, beverages, cattle feeds or the like. (See Kosikowski, *Supra*, p. 446).

Having now generally described this invention, the same will be further understood by reference to certain specific examples which are provided herein for purposes of illustration only and are not intended to limiting unless otherwise specified.

Example 1

Five Holstein cows were immunized against *Escherichia coli* (American Type Culture Strain No. 13076). The primary immunization was accomplished by intramuscular injection of a vaccine containing heat-killed *E. coli* cells suspended in physiological saline. The concentration of bacterial cells was $4 \times 10^8$/ml. A dose of

5 ml ($20 \times 10^8$) cells was injected i.m. once weekly for four consecutive weeks. Milk collected during the fifth week was tested for the presence of antibodies against *E. coli*. The presence of antibody against *E. coli* was determined using a micro-agglutination procedure. This procedure involves reacting different dilutions of milk whey with a fixed concentration of *E. coli* bacterial cells suspended in buffer. The presence of antibodies in the milk causes agglutination of the bacterial cells. The milk is diluted in a serial fashion and there comes a point when the concentration of antibodies is too low to cause the agglutination reaction. The maximum dilution which causes agglutination is the antibody titer. The presence of high antibody concentration in milk is an indication that the immunization procedure causes sensitization of the cow's immune system against the antigen. Table 1 compares the antibody titer against *E. coli* in the five cows before and after primary immunization.

TABLE 1
Milk antibody titer in 5 cows before and after
immunization against *E. coli*

| Cow No. | Before immunization | After immunization |
|---------|---------------------|--------------------|
| 1 | 0 | 640 |
| 2 | 0 | 1280 |
| 3 | 0 | 5000 |
| 4 | 0 | 1280 |
| 5 | 0 | 10,000 |

In each case there was a significant increase in the milk titer against *E. coli* following immunization. From this we conclude that the immunization caused sensitization of the cow against the *E. coli*. Having induced a state of sensitivity, the cows were given booster injections of the same dose of antigen every 14 days, thus establishing and maintaining a period of anti-inflammatory factor producing state during which time the milk was collected daily and processed to obtain anti-inflammatory skimmed powdered milk.

The anti-inflammatory skimmed powdered milk induced by the method outlined above was tested for anti-inflammatory properties using the rat paw test.

Ten adult white rats were fed 10 mg of anti-inflammatory milk suspended in 20 ml of $H_2O$ daily for five consecutive days. On the fifth day the right rat paw was injected with 1/10 of a ml of 1% carrageenan saline solution, and 24 hours after injection the rats sacrificed and the right and left paws amputated and weighed. The mean weight of the left paw in the experimental group was compared to the mean weight of the right paw using the student's paired T test to determine if a statistically significant difference exists between right and left paws. The identical experiment was conducted in a second group of rats using the same quantity of normal skimmed milk instead of the anti-inflammatory milk. A third group of rats was fed just water. Results from this experiment are summarized in Table 2.

TABLE 2
Rat paw test results—Example 1

| Rat No. | Group 1—Fed anti-inflammatory milk | | |
| | Right paw wt. (g) | Left paw wt. (g) | Difference |
| --- | --- | --- | --- |
| 1 | 1.87 | 1.66 | 0.21 |
| 2 | 1.61 | 1.61 | 0.00 |
| 3 | 1.68 | 1.65 | 0.03 |
| 4 | 1.95 | 1.87 | 0.08 |
| 5 | 1.82 | 1.77 | 0.05 |
| 6 | 1.83 | 1.74 | 0.09 |
| 7 | 1.84 | 1.67 | 0.17 |
| 8 | 1.63 | 1.68 | 0.05 |
| 9 | 1.75 | 1.70 | 0.05 |
| 10 | 1.78 | 1.78 | 0.00 |
| Mean±S.D. | 1.78±0.11 | 1.71±0.08 | 0.07±0.07 |

| Rat No. | Group 2—Fed normal milk | | |
| | Right paw wt. (g) | Left paw wt. (g) | Difference |
| --- | --- | --- | --- |
| 1 | 1.68 | 1.48 | 0.20 |
| 2 | 1.82 | 1.67 | 0.15 |
| 3 | 1.69 | 1.60 | 0.09 |
| 4 | 1.95 | 1.69 | 0.26 |
| 5 | 1.96 | 1.74 | 0.22 |
| 6 | 2.22 | 1.77 | 0.45 |
| 7 | 1.78 | 1.49 | 0.29 |
| 8 | 1.75 | 1.60 | 0.15 |
| 9 | 1.78 | 1.70 | 0.08 |
| 10 | 2.12 | 1.61 | 0.51 |
| Mean±S.D. | 1.88±0.18 | 1.64±0.10 | 0.24±0.14 |

6

# 0 064 103

| | Group 3—Fed water | | |
|---|---|---|---|
| Rat No. | Right paw wt. (g) | Left paw wt. (g) | Difference |
| 1 | 1.80 | 1.48 | 0.32 |
| 2 | 1.78 | 1.55 | 0.23 |
| 3 | 1.75 | 1.63 | 0.12 |
| 4 | 1.89 | 1.67 | 0.22 |
| 5 | 2.02 | 1.77 | 0.25 |
| 6 | 2.95 | 1.73 | 0.22 |
| 7 | 1.78 | 1.65 | 0.13 |
| 8 | 1.87 | 1.60 | 0.27 |
| 9 | 1.88 | 1.62 | 0.26 |
| 10 | 1.90 | 1.77 | 0.13 |
| Mean±S.D. | 1.86±0.09 | 1.65±0.09 | 0.22±0.07 |

The mean weight of the right hind paw in rats fed $H_2O$ was significantly greater than the mean weight of the left paw, due to swelling produced in the right paw by the injection of carrageenan. The identical result occurred in the group of rats that were fed normal powdered skimmed milk, whereas in rats fed anti-inflammatory skimmed powdered milk, there was no significant difference between the mean weight of the right and left paws. These results clearly demonstrate that the product blocks the inflammatory response.

Comparative Example 1

The process of Example 1 was repeated except that the dosage of *E. coli* cells used for booster injections was $1 \times 10^3$. The cow was in an immune state but not in an anti-inflammatory factor producing state, as demonstrated by antibodies in the milk against *E. coli* and by the non-effectiveness of the milk obtained therefrom to block rat paw inflammation.

Example 2

The identical experiment as described in Example 1 was repeated using the same dosage but utilizing a different bacterial species, i.e., *Salmonella enteritidis*, as the selected antigen for the induction of the sensitivity. The results of the anti-inflammatory tests performed on the milk produced using this antigen were positive.

Example 3

The identical experiment as described in Example 1 was undertaken with the exception that a polyvalent vaccine comprised of the bacterial strains listed below in Table 3 was used as the selected antigen. The different bacterial strains were combined by mixing equal weights of the lyophilized bacterial cells and diluting the mixture in saline to obtain a concentration identical to that used in Example 1. Results of the anti-inflammatory tests on milk produced using this selected antigen were positive.

7

**0 064 103**

TABLE 3
Bacterial antigens

| Organism | *ATCC No. |
|---|---|
| *Staphylococcus aureus* | 11631 |
| *Staphylococcus epidermidis* | 155 |
| *Streptococcus pyogenes,* A. Type 1 | 8671 |
| *Streptococcus pyogenes,* A. Type 3 | 10389 |
| *Streptococcus pyogenes,* A. Type 5 | 12347 |
| *Streptococcus pyogenes,* A. Type 8 | 12389 |
| *Streptococcus pyogenes,* A. Type 12 | 11434 |
| *Streptococcus pyogenes,* A. Type 14 | 12972 |
| *Streptococcus pyogenes,* A. Type 18 | 12357 |
| *Streptococcus pyogenes,* A. Type 22 | 10403 |
| *Aerobacter aerogenes* | 884 |
| *Escherichia coli* | 26 |
| *Salmonella enteritidis* | 13076 |
| *Pseudomonas aeruginosa* | 7700 |
| *Klebsiella pneumoniae* | 9590 |
| *Salmonella typhimurium* | 13311 |
| *Haemophilus influenzae* | 9333 |
| *Streptococcus viridans* | 6249 |
| *Proteus vulgaris* | 13315 |
| *Shigella dysenteriae* | 11835 |
| *Streptococcus,* Group B | |
| *Diplococcus pneumoniae* | |
| *Streptococcus mutans* | |
| *Corynebacterium, Acne,* Types 1 & 2 | |

* American Type Culture Collection, 12301 Parklawn Drive, Rockville, Maryland 20852.

It should be noted here that the milk produced in this Example is identical to that produced in a preferred embodiment of the invention disclosed in U.K. Patent Specification No. 2013691 (published 15th August, 1979). This co-pending application discloses that the milk of the present Example also has highly beneficial anti-arthritic properties. The anti-arthritic properties of the milk are due to the presence of antibodies therein which decrease the levels of Rheumatoid Factor (RF) in the blood of arthritic patients. It is of course possible that the antibodies present in the milk and the anti-inflammatory factor of the same milk, act in a synergistic manner. The factor may decrease joint inflammation while the antibodies may decrease the level of RF.

8

**0 064 103**

It must be noted however that (a) antibody isolated from this milk has no anti-inflammatory effects; (b) non-arthritic conditions such as allergies or tennis elbow can be successfully treated with the milk; (c) there is no mechanism to explain how an anti-inflammatory factor can decrease the level of RF in the blood. These facts indicate that the anti-inflammatory and anti-arthritic properties of the milk are caused by different factors, and that anti-inflammatory factor is not an antibody.

Example 4

The identical experiment was undertaken as per Example 1 with the exception that a protein hormone (hCG) was used in lieu of bacterial cells as the selected antigen. For this experiment 10 mg of chorionic gonadotropin hormone was dissolved in 5 mls of physiological saline to formulate the vaccine used for both primary immunization and subsequent booster immunizations according to the same sequence as described for Example 1. The results from anti-inflammatory tests on the milk produced using this selected antigen were positive.

Example 5

The identical procedure as per Example 1 was performed with exception that an animal cell antigen was used in lieu of the bacterial antigen for sensitization of the cow and subsequent booster injections. For this experiment, 10 mg of rat mammary tumor tissue was used to prepare the vaccine for the primary and booster injections. The vaccine was prepared by homogenizing 10 mg of tumor tissue obtained from mammary tumors collected from rats. The tumors were thoroughly homogenized and the resulting material was injected into cows using the same primary and booster immunization schedule as described in Example 1. The results of the anti-inflammatory tests in milk produced using this selected antigen for the induction of sensitivity and maintenance of the hyperimmune state were positive.

Example 6

A strain of *Streptococcus mutans* was cultured in accordance with established techniques. Cultures of *S. mutans* AHT (serological group a), BHT (group b), 10449 (group c) and 6715 (group d) were grown in dialyzed tryptose medium. The cells were harvested by centrifugation at $4000 \times G$ and washed five times with 0.1 M phosphate buffered saline, pH 7.0. The cells were inactivated by heating at 60°C for 30 minutes and resuspended to a final concentration of *S. mutans* AHT, BHT, 10449 and 6715 at $5 \times 10^8$ cells/ml. This preparation was used to immunize two cows. Each cow was immunized on two separate occasions with fresh antigen from all four groups of *S. mutans* (groups a, b, c and d). A cow was then immunized in accordance with the established techniques of this invention to generate a milk product. Following immunization, blood samples of the cow were taken until the serum anti-body titre reached its highest level, then the milk was collected. The milk itself was then dried and powdered, again in accordance with established techniques to produce a powdered milk having positive anti-inflammatory effects.

It should be noted here that the milk produced in this Example is identical to the caries-inhibiting milk disclosed by *Beck* (the present inventor) in GB—A—1,505,513.

The preceding examples establish an important principle of the invention; i.e., that the nature of the antigen used to induce sensitivity and to maintain the anti-inflammatory factor producing state can be varied. Although it is possible to maintain an anti-inflammatory factor producing state utilizing a single antigen as demonstrated in Examples 1 and 2, the likelihood of tolerance developing through the use of a single antigen is much greater than that of multiple antigens as per Example 3. The same rationale would apply to antigens of different types including hormones, viruses, proteins, toxins, or the like.

Having now generally described this invention it will become readily apparent to one skilled in the art that many changes and modifications can be made thereto without affecting the scope thereof as defined in the following Claims.

**Claims**

1. A method of obtaining an anti-inflammatory milk or anti-inflammatory product derived therefrom which comprises:—

selecting an antigen or mixture of antigens capable of inducing a state of immune sensitivity in a bovid,

sensitizing the bovid by primary immunization with said antigen(s),

testing the serum of the bovid to confirm sensitivity induction, and

administering to the bovid booster doses of said antigen(s) on a periodic basis to maintain a hyperimmune state,

characterised in that milk produced by the bovid is tested for anti-inflammatory activity and milk is collected for the purposes of the method from the bovid only during the period said test confirms that the milk contains an anti-inflammatory factor.

2. A method of obtaining an anti-inflammatory milk or an anti-inflammatory product derived therefrom which comprises:—

testing milk from a bovid being maintained in a hyperimmune state and

collecting milk from the bovid during said hyperimmune state,

characterised in that milk produced by the bovid is tested for anti-inflammatory activity and the milk is

9

collected for the purpose of the method from the bovid only during the period said test confirms that the milk contains an anti-inflammatory factor.

3. A method of obtaining an anti-inflammatory milk or anti-inflammatory product derived therefrom which comprises:—

selecting an antigen or mixture of antigens capable of inducing a state of immune sensitivity in a bovid, sensitizing the bovids by primary immunization with said antigen(s),

testing the serum of the bovids to confirm sensitivity induction, and

administering to the bovids booster doses of said antigen(s) on a periodic basis to maintain a hyperimmune state,

characterised in that milk produced by the bovid is tested for anti-inflammatory activity, bovids giving milk failing said anti-inflammatory test are subjected to readministration of said booster doses or rejected for the purposes of the method, and milk is collected in the method from the bovid only during the period said test confirms that the milk contains an anti-inflammatory factor.

4. A method as claimed in Claim 2, wherein said hyperimmune state has been induced by sensitizing the bovid by primary immunization with an antigen or mixture of antigens and the hyperimmune state has been maintained by booster doses of said antigen(s) on a periodic basis.

5. A method as claimed in Claim 1, Claim 3 or Claim 4, wherein the antigen is a polyvalent antigen.

6. A method as claimed in Claim 5, wherein the polyvalent antigen is comprised of:—

| Organism | American type culture collection No. |
|---|---|
| *Staphylococcus aureus* | 11631 |
| *Staphylococcus epidermidis* | 155 |
| *Streptococcus pyogenes*, A. Type 1 | 8671 |
| *Streptococcus pyogenes*, A. Type 3 | 10389 |
| *Streptococcus pyogenes*, A. Type 5 | 12347 |
| *Streptococcus pyogenes*, A. Type 8 | 12389 |
| *Streptococcus pyogenes*, A. Type 12 | 11434 |
| *Streptococcus pyogenes*, A. Type 14 | 12972 |
| *Streptococcus pyogenes*, A. Type 18 | 12357 |
| *Streptococcus pyogenes*, A. Type 22 | 10403 |
| *Aerobacter aerogenes* | 884 |
| *Escherichia coli* | 26 |
| *Salmonella enteritidis* | 13076 |
| *Pseudomonas aeruginosa* | 7700 |
| *Klebsiella pneumoniae* | 9590 |
| *Salmonella typhimurium* | 13311 |
| *Haemophilus influenzae* | 9333 |
| *Streptococcus viridans* | 6249 |
| *Proteus vulgaris* | 13315 |
| *Shigella dysenteriae* | 11835 |
| *Streptococcus*, Group B | |
| *Diplococcus pneumoniae* | |
| *Streptococcus mutans* | |
| *Corynebacterium, Acne*, Types 1 & 2 | |

7. A method as claimed in Claim 1, Claim 3 or Claim 4, wherein the antigen is *E. coli*.

8. A method as claimed in Claim 1, Claim 3 or Claim 4, wherein the antigen is *Salmonella enteritidis*.

9. A method as claimed in Claim 1, Claim 3 or Claim 4, wherein the antigen is chorionic gonadotropin hormone.

10. A method as claimed in Claim 1, Claim 3 or Claim 4, wherein the antigen is rat mammary tumor tissue vaccine.

11. A method as claimed in Claim 1, Claim 3 or Claim 4, wherein the antigen is *Streptococcus mutans*.

12. A method as claimed in any one of the preceding claims, wherein the bovid is a cow.

13. A method as claimed in any one of the preceding claims, wherein said primary immunization and/or hyperimmune state are/is produced by respective doses of $10^6$ to $10^{20}$ cells.

14. A method as claimed in Claim 13, wherein said dose is $10^8$ to $10^{10}$ cells.

15. A method as claimed in Claim 14, wherein said dose is $2 \times 10^8$ cells.

16. A method as claimed in any one of the preceding claims, wherein the booster doses maintaining the hyperimmune state are equal to or greater than 50% of the dose producing primary immunization.

11

17. A method as claimed in Claim 5, Claim 6 or any one of Claims 12 to 16 when appendant to Claim 5 or Claim 6, wherein primary immunization is confirmed by testing for the presence of agglutinating antibodies in the serum of the bovid before and after challenge with the polyvalent antigen.

18. A method as claimed in any one of the preceding claims, wherein the hyperimmune state is confirmed by testing the milk for anti-inflammatory activity by a rat paw anti-inflammatory test.

19. A method as claimed in any one of the preceding claims, wherein the collected milk is pasteurized at a temperature not exceeding 140°C.

20. A method as claimed in Claim 19, wherein, following pasteurization, fat is removed from the milk.

21. A method as claimed in Claim 20, wherein the fat free milk is concentrated under vacuum at a sufficiently low temperature not to destroy the anti-inflammatory factor, and then spray dried.

22. A method as claimed in Claim 20, wherein the fat free milk is treated with acid at about room temperature to bring it to pH 4.2 to 4.6 and the casein separated after precipitation thereof to provide an acid-whey fraction containing the anti-inflammatory factor.

**Patentansprüche**

1. Verfahren zur Herstellung einer entzündungsverhindernden Milch oder eines enzündungsverhindernden, davon abgeleiteten Produktes, welches umfasst:
Auswahl eines Antigens oder einer Mischung von Antigenen, die fähig sind, einen Zustand immuner Sensitivität bei einem Boviden zu induzieren;
Sensitivierung des Boviden durch eine primäre Immunisierung mit dem (den) genannten Antigen(en);
Test des Serums des Boviden zur Bestätigung der Sensitivitätinduktion; und
Verabreichung von Zusatzdosen des (der) genannten Antigen(e) an den Boviden auf periodischer Basis, um einen hyperimmunen Zustand aufrecht zu erhalten; dadurch gekennzeichnet, dass die vom Boviden produzierte Milch auf entzündungsverhindernde Aktivität geprüft wird und die Milch zum Zwecke des Verfahrens vom Boviden nur während der Periode gesammelt wird, in welcher der genannte Test bestätigt, dass die Milch einen entzündungsverhindernden Faktor enthält.

2. Verfahren zur Herstellung einer entzündungsverhindernden Milch oder eines entzündungsverhindernden, davon abgeleiteten Produktes, welches umfasst:
Testen der Milch eines Boviden, der in einem hyperimmunen Zustand gehalten wird und Sammeln der Milch des Boviden während dem genannten hyperimmunen Zustand; dadurch gekennzeichnet, dass die durch den Boviden produzierte Milch auf entzündungshindernde Aktivität getestet wird und die Milch zum Zwecke des Verfahrens nur während derjenigen Periode vom Boviden gesammelt wird, wenn der Test bestätigt, dass die Milch einen entzündungsverhindernden Faktor enthält.

3. Verfahren zur Herstellung einer entzündungsverhindernden Milch oder eines entzündungsverhindernden, davon abgeleiteten Produktes, welches umfasst:
Auswahl eines Antigens oder einer Mischung von Antigenen, die fähig sind, bei einem Boviden einen Zustand immuner Sensitivität zu induzieren;
Sensitivierung der Boviden durch eine primäre Immunisierung mit dem (den) genannten Antigen(en);
Test des Serums des Boviden zur Bestätigung der Sensitivitätsinduktion; und
Verabreichung von Zusatzdosen des (der) genannten Antigen(e) an den Boviden auf periodischer Basis zur Aufrechterhaltung eines hyperimmunen Zustandes; dadurch gekennzeichnet, dass Milch, welche durch den Boviden produziert wird, auf entzündungsverhindernde Aktivität getestet wird, die Boviden, welche den entzündungsverhindernden Test nicht bestehen, einer Wiederverabreichung der genannten Zusatzdosen unterworfen werden oder für die Zwecke des Verfahrens zurückgewiesen werden, und die Milch im Verfahren von den Boviden nur während derjenigen Periode gesammelt wird, von welcher die Tests bestätigen, dass die Milch einen entzündungsverhindernden Faktor enthält.

4. Verfahren gemäss Anspruch 2, worin der genannte hyperimmune Zustand induziert wurde, indem der Bovid durch eine primäre Immunisierung mit einem Antigen oder einer Mischung von Antigenen sensitiviert wird und der hyperimmune Zustand durch Zusatzdosen des (der) genannten Antigen(e) auf periodischer Basis aufrechterhalten worden ist.

5. Verfahren gemäss Anspruch 1, Anspruch 3 oder Anspruch 4, worin das Antigen ein polyvalentes Antigen ist.

6. Verfahren gemäss Anspruch 5, worin das polyvalente Antigen folgendes enthält:

# 0 064 103

| Organismus | "American Type Culture Collection No" |
|---|---|
| *Staphylococcus aureus* | 11631 |
| *Staphylococcus epidermidis* | 155 |
| *Streptococcus pyogenes,* A. Typ 1 | 8671 |
| *Streptococcus pyogenes,* A. Typ 3 | 10389 |
| *Streptococcus pyogenes,* A. Typ 5 | 12347 |
| *Streptococcus pyogenes,* A. Typ 8 | 12389 |
| *Streptococcus pyogenes,* A. Typ 12 | 11434 |
| *Streptococus pyogenes,* A. Typ 14 | 12972 |
| *Streptococcus pyogenes,* A. Typ 18 | 12357 |
| *Streptococcus pyogenes,* A. Typ 22 | 10403 |
| *Aerobacter aerogenes* | 884 |
| *Escherichia coli* | 26 |
| *Salmonella enteritidis* | 13076 |
| *Pseudomonas aeruginosa* | 7700 |
| *Klebsiella pneumoniae* | 9590 |
| *Salmonella typhimurium* | 13311 |
| *Haemophilus influenzae* | 9333 |
| *Streptococcus viridans* | 6249 |
| *Proteus vulgaris* | 13315 |
| *Shigella dysenteriae* | 11835 |
| *Streptococcus,* Gruppe B | |
| *Diplococcus pneumoniae* | |
| *Streptococcus mutans* | |
| *Corynebacterium, Acne,* Typen 1 & 2 | |

7. Verfahren gemäss Anspruch 1, Anspruch 3 oder Anspruch 4, worin das Antigen E. coli ist.

8. Verfahren gemäss Anspruch 1, Anspruch 3 oder Anspruch 4, worin das Antigen Salmonella enteritidis ist.

9. Verfahren gemäss Anspruch 1, Anspruch 3 oder Anspruch 4, worin das Antigen das Chorion-Gonadotropin-Hormon ist.

10. Verfahren gemäss Anspruch 1, Anspruch 3 oder Anspruch 4, worin das Antigen Impfstoff von Tumorgewebe der Mamma der Ratte ist.

11. Verfahren gemäss Anspruch 1, Anspruch 3 oder Anspruch 4, worin das Antigen Streptococcus mutans ist.

12. Verfahren gemäss einem der vorhergehenden Ansprüche, worin der Bovid eine Kuh ist.

13. Verfahren gemäss einem der vorhergehenden Ansprüche, worin die genannte primäre Immunisierung und/oder der hyperimmune Zustand durch entsprechende Dosen von $10^6$—$10^{20}$ Zellen produziert sind/ist.

14. Verfahren gemäss Anspruch 13, worin die genannte Dosis $10^8$—$10^{10}$ Zellen umfasst.

**0 064 103**

15. Verfahren gemäss Anspruch 14, worin die genannte Dosis $2\times10^8$ Zellen umfasst.

16. Verfahren gemäss einem der vorhergehenden Ansprüche, worin die Zusatzdosen, welche den hyperimmunen Zustand aufrecht erhalten, gleich oder grösser als 50% der Dosis sind, welche die primäre Immunisierung erzeugt.

17. Verfahren gemäss Anspruch 5, Anspruch 6 oder einem der Ansprüche 12—16, wenn abhängig von Anspruch 5 oder 6, worin die primäre Immunisierung bestätigt wird, durch Testen der Gegenwart von agglutinierenden Antikörpern im Serum des Boviden vor oder nach der Herausforderung mit dem polyvalenten Antigen.

18. Verfahren gemäss einem der vorhergehenden Ansprüche, worin der hyperimmune Zustand durch Testen der Milch auf entzündungsverhindernde Aktivität durch einen entzündungsverhindernden Test der Rattenpfote bestätigt wird.

19. Verfahren gemäss einem der vorhergehenden Ansprüche, worin die gesammelte Milch bei einer Temperatur, welche 140°C nicht überschreitet, pasteurisiert wird.

20. Verfahren gemäss Anspruch 19, worin im Anschluss an die Pasteurisierung der Milch das Fett entzogen wird.

21. Verfahren gemäss Anspruch 20, worin die fettfreie Milch unter Vakuum konzentriert wird, bei einer genügend tiefen Temperatur, um den entzündungsverhindernden Faktor nicht zu zerstören, und dann sprühgetrocknet wird.

22. Verfahren gemäss Anspruch 20, worin die fettfreie Milch bei etwa Zimmertemperatur mit Säure behandelt wird, um den pH-Wert auf 4,2—4,6 zu bringen, und das Kasein nach seiner Ausfällung abgetrennt wird, um eine Säuremolkenfraktion bereitzustellen, welche den entzündungsverhindernden Faktor enthält.

**Revendications**

1. Procédé d'obtention d'un lait anti-inflammatoire ou d'un produit anti-inflammatoire provenant de celui-ci, qui consiste:

— à choisir un antigène ou un mélange d'antigènes capable de provoquer l'apparition d'un état de sensibilité immune chez un bovidé,
— à sensibiliser le bovidé par immunisation primaire avec ce ou ces antigènes,
— à essayer le sérum du bovidé pour confirmer l'induction d'une sensiblité, et
— à administrer au bovidé des doses de rappel de ce ou ces antigènes sur une base périodique pour maintenir un état hyperimmun,

caractérisé en ce que le lait produit par le bovidé est soumis à un essai d'activité anti-inflammatoire et en ce que le lait est recueilli du bovidé, pour l'utilisation dans le procédé, seulement au cours de la période pendant laquelle cet essai confirme que le lait contient un facteur anti-inflammatoire.

2. Procédé d'obtention d'un lait anti-inflammatoire ou d'un produit anti-inflammatoire provenant de celui-ci, qui consiste:

— à essayer le lait d'un bovidé maintenu dans un état hyperimmun, et
— à recueillir du lait du bovidé au cours de cet état hyperimmun,

caractérisé en ce que le lait produit par le bovidé est soumis à un essai d'activité anti-inflammatoire et en ce que le lait est recueilli du bovidé pour l'utilisation dans le procédé, seulement au cours de la période pendant laquelle cet essai confirme que le lai contient un facteur anti-inflammatoire.

3. Procédé d'obtention d'un lait anti-inflammatoire ou d'un produit anti-inflammatoire provenant de celui-ci, qui consiste:

— à choisir un antigène ou un mélange d'antigènes capable de provoquer un état de sensibilité immune chez un bovidé,
— à sensibiliser les bovidés par immunisation primaire avec ce ou ces antigènes,
— à essayer le sérum des bovidés pour confirmer l'induction d'une sensibilité, et
— à administrer aux bovidés des doses de rappel de ce ou ces antigène(s) sur une base périodique pour maintenir un état hyperimmun;

caractérisé en ce que le lait produit par le bovidé est soumis à un essai d'activité anti-inflammatoire, les bovidés donnant un lait ne satisfaisant pas à cet essai anti-inflammatoire étant soumis à une réadministration de ces doses de rappel ou rejetés pour l'utilisation dans le procédé, et en ce que le lait est recueilli du bovidé dans le procédé seulement au cours de la période pendant laquelle cet essai confirme que le lait contient un facteur anti-inflammatoire.

4. Procédé suivant la revendication 2, dans lequel cet état hyperimmun a été induit en sensibilisant le bovidé par immunisation primaire avec un antigène ou un mélange d'antigènes et l'état hyperimmun a été maintenu par des doses de rappel de ce ou ces antigènes sur une base périodique.

14

5. Procédé suivant la revendication 1, la revendication 3 ou la revendication 4, dans lequel l'antigène est un antigène polyvalent.

6. Procédé suivant la revendication 5, dans lequel l'antigène polyvalent est constitué de:

| Organisme | No. de l'American Type Culture Colection |
|---|---|
| Staphylococcus aureus | 11631 |
| Staphylococcus epidermidis | 155 |
| Streptococcus pyogenes, A. Type 1 | 8671 |
| Streptococcus pyogenes, A. Type 3 | 10389 |
| Streptococcus pyogenes, A. Type 8 | 12389 |
| Streptococcus pyogenes, A. Type 12 | 11434 |
| Streptococcus pyogenes, A. Type 14 | 12972 |
| Streptococcus pyogenes, A. Type 18 | 12357 |
| Streptococcus pyogenes, A. Type 22 | 10403 |
| Aerobacter aerogenes | 884 |
| Escherichia coli | 26 |
| Salmonella enteritidis | 13076 |
| Pseudomonas aeruginosa | 7700 |
| Klebsiella pneumoniae | 9590 |
| Salmonella typhimurium | 13311 |
| Haemophilus influenzae | 9333 |
| Streptococcus viridans | 6249 |
| Proteus vulgaris | 13315 |
| Shigella dysenteriae | 11835 |
| Streptococcus, Groupe B | |
| Diplococcus pneumoniae | |
| Streptococcus mutans | |
| Corynebacterium, Acne, Types 1 & 2 | |

7. Procédé suivant la revendication 1, la revendication 3 ou la revendication 4, dans lequel l'antigène est E. coli.

8. Procédé suivant la revendication 1, la revendication 3 ou la revendication 4, dans lequel l'antigène est Salmonella enteritidis.

9. Procédé suivant la revendication 1, la revendication 3 ou la revendication 4, dans lequel l'antigène est la gonadotrophine chorionique.

10. Procédé suivant la revendication 1, la revendication 3 ou la revendication 4, dans lequel l'antigène est du vaccin de tissus de tumeur mammaire du rat.

11. Procédé suivant la revendication 1, la revendication 3 ou la revendication 4, dans lequel l'antigène est Streptococcus mutans.

12. Procédé suivant l'une quelconque des revendications précédentes, dans lequel le bovidé est une vache.

13. Procédé suivant l'une quelconque des revendications précédentes, dans lequel cet état d'immunisation primaire et/ou cet état d'hyperimmun sont produits par des doses respectives de $10^6$ à $10^{20}$ cellules.

14. Procédé suivant la revendication 13, dans lequel cette dose est de $10^8$ à $10^{10}$ cellules.

15. Procédé suivant la revendication 14, dans lequel cette dose est de $2\times10^8$ cellules.

16. Procédé suivant l'une quelconque des revendications précédentes, dans lequel les doses de rappel maintenant l'état hyperimmun sont égales ou supérieures à 50% de la dose produisant l'immunisation primaire.

17. Procédé suivant la revendication 5, la revendication 6 ou l'une quelconque des revendications 12 à 16 lorsqu'elles sont rattachées à la revendication 5 ou la revendication 6, dans lequel l'immunisation primaire est confirmée par essai de la présence d'anticorps agglutinant dans le sérum de bovidé avant et après provocation par l'antigène polyvalent.

18. Procédé suivant l'une quelconque des revendications précédentes, dans lequel l'état hyperimmun est confirmé en soumettant le lait à un essai d'activité anti-inflammatoire par un essai anti-inflammatoire sur la patte de rat.

19. Procédé suivant l'une quelconque des revendications précédentes, dans lequel le lait recueilli est pasteurisé à une température ne dépassant pas 140°C.

20. Procédé suivant la revendication 19, dans lequel, après pasteurisation, la graisse est éliminée du lait.

21. Procédé suivant la revendication 20, dans lequel le lait exempt de graisse est concentré sous vide à une température suffisamment basse pour ne pas détruire le facteur anti-inflammatoire, et séché par pulvérisation.

22. Procédé suivant la revendication 20, dans lequel le lait exempt de graisse est traité par un acide aux environs de la température ambiante pour amener son pH à 4,2 à 4,6 et la caséine est séparée après sa précipitation pour fournir une fraction de petit lait acide contenant le facteur anti-inflammatoire.